# FASCICULE DE BREVET EUROPEEN

(11) **EP 2 200 965 B1**
(45) Date de publication et mention de la délivrance du brevet: **10.10.2018**
(21) Numéro de dépôt: 08841849.6
(22) Date de dépôt: 21.10.2008
(51) Int. Cl.: C07C 69/34, C07C 69/40, C07C 69/42, C07C 69/44, C11D 7/26, C23G 5/032, C09K 3/32, C09D 9/00, C11D 7/50

(54) **DIESTERS D'ACIDES DICARBOXYLIQUES, PROCEDES DE PREPARATION ET UTILISATIONS**
DICARBOXYLSÄUREDIESTER, VERFAHREN ZU IHRER HERSTELLUNG UND VERWENDUNGEN DAVON
DICARBOXYLIC ACID DIESTERS, METHODS FOR PREPARING SAME AND USES THEREOF

(30) Priorité: 22.10.2007 FR 0707367
(43) Date de publication de la demande: 30.06.2010
(73) Titulaire: Rhodia Opérations, 93306 Aubervilliers (FR)
(72) Inventeur: JACQUOT, Roland, F-69340 Francheville (FR); MARION, Philippe, F-69390 Vernaison (FR); ZANETTO, Jean-Emille, F-75009 Paris (FR); JENTZER, Olivier, F-69390 Vourles (FR); DEROO, Sophie, F-94240 L'Hay les Roses (FR); LABARRE, Dominique, F-92200 Neuilly sur Seine (FR)
(74) Mandataire: Cardon, Flavie
(86) Numéro de dépôt international: PCT/EP2008/064179
(87) Numéro de publication internationale: WO 2009/053347

(56) Documents cités:
- EP-A- 0 743 358
- WO-A1-2007/101929
- FR-A- 2 878 157
- GB-A- 1 041 334
- US-A- 4 110 626
- US-A- 4 904 814
- US-A1- 2002 183 575
- HU GUOQIANG ET AL: "Synthesis of Saturated Fatty Glutarate Using Heteropoly Acid as Catalyst" FUSHUN-SHIYOU-XUEYUAN-XUEBAO = JOURNAL OF FUSHUN PETROLEUM INSTITUTE,, vol. 19, no. 2, 1 juin 1999 (1999-06-01), pages 24-28, XP009102723 ISSN: 1005-3883
- COHEN, MURPHY, O'REAR, RAVNER, ZISMAN: "Aliphatic esters" INDUSTRIAL AND ENGINEERING CHEMISTRY, vol. 45, 1953, pages 1766-1775, XP002487117
- WESLEY RIGG, GISSER: JOURNAL OF THE AMERICAN CHEMICAL SOCIETY, vol. 75, 1953, pages 1415-1420, XP002487118
- JAMES, BRYAN: JOURNAL OF ORGANIC CHEMISTRY, vol. 23, 1958, pages 1225-1227, XP002487119
- KÖNIG ET BENECKE: JOURNAL OF CHROMATOGRAPHY, vol. 195, 1980, pages 929-296, XP002487120
- ET ALBIOORGANIC &: "Synthesis and biological evaluation of aroylguanidines related to amiloride as inhibitors of the human platelet Na<+>/H<+> exchanger" ICINAL CHEMISTRY, ELSEVIER SCIENCE LTD, GB, vol. 10, no. 6, 1 janvier 2002 (2002-01-01), pages 1793-1804, XP002429666 ISSN: 0968-0896
- XUE CUIHUA ET AL: "TRANSFORMATION OF AMIDES INTO ESTERS BY THE USE OF CHLOROTRIMETHYLSILANE" JOURNAL OF THE CHINESE CHEMICAL SOCIETY, XX, XX, vol. 51, no. 2, 1 janvier 2004 (2004-01-01), pages 359-362, XP008077858 ISSN: 0009-4536
- Rhodia: "Solvents Rhodiasolv", , 1 September 2003 (2003-09-01), Retrieved from the Internet: URL:http://www.rhodia.com/en/binaries/Nove care_Rhodiasolv_brochure_2008_EN.pdf [retrieved on 2012-05-16]

## Description

La présente invention a pour objet de nouveaux diesters d'acides dicarboxyliques, pouvant notamment être obtenus à l'aide d'huile de fusel. Ces composés peuvent notamment être utiles comme solvants.

Il a été décrit des composés de type diesters d'acides dicarboxyliques. Il a été par ailleurs décrit des composés dérivant de l'huile de fusel. Certains usages et certaines propriétés de ces composés ont été décrits.

Le document "Esterification Reaction of Oleic Acid with a fusel oil fraction for production of lubricating oil", Özgülsün et al., JAOCS, Vol. 77, no.1 (2000), p 105, décrit des produits d'estérification de mono-acides.

Le document FR 2878157 décrit l'acétate d'huile de fusel, et son utilisation dans des vernis à ongles, à titre de solvant à bas COV.

Le document "The use of Egyptian Fusel Oil for the préparation of some plasticizers compatible with polyvinyl Chloride", Chuiba et al., Indian Journal of Technology, Vol. 23, August 1985, pp 309-311, décrit un produit de réaction d'une huile de fusel ayant un point d'ébullition de 110 à 136°C et d'acide adipique ou sébacique pur. Les propriétés plastifiantes qui ont été présentées pour ces composés sont médiocres par rapport à celles qui ont été présentées pour les autres.

Par ailleurs il existe dans le commerce des solvants de type diester d'acides dicarboxyliques ("dibasic esters" en anglais), qui sont des produits d'estérification d'un mélange d'acide adipique, glutarique et succinique, avec un alcool methylique, éthylique ou di-isobutylique. On connait notamment les produits commercialisés par Rhodia: Rhodiasolv® RPDE qui est un diester méthylique, le Rhodiasolv® DEE qui est un diester éthylique, le Rhodiasolv® DIB qui est un diester isobutylique. (Brochure, Rhodia-Novecare, Rhodiasolv DIB, Septembre 2003). Ces produits présentent un excellent profile écologique, une excellente biodégradabilité, et un excellent profile de sécurité, et proviennent de processus de fabrication optimisant les ressources et diminuant l'impact environnemental. Leurs utilisations sont toutefois limitées, et il existe un besoin pour des produits présentant une activité supérieure au moins sur certaines matières. Il existe notamment un besoin pour d'autres produits présentant un aussi bon profile, voir même meilleur.

L'invention répond à ce besoin en proposant une composition de matière à base de diester(s) d'acide(s) dicarboxylique(s) de formule (I):

R¹-OOC-A-COO-R² (I)

où
- les groupes R¹ et R², identiques ou différents, représentent un groupe alkyle, aryle, alkyaryle, ou arylalkyle, linéaire ou branché, cyclique ou non cyclique, en C₁-C₂₀
caractérisé(e) en ce que:
- au moins une partie des groupes R¹ et/ou R² sont des groupes alkyl branchés comprenant 5 atomes de carbone, et/ou
- au moins une partie des groupes R¹ et/ou R² proviennent d'huile de fusel,
- la composition de matière comprenant :
- de 1% à 20% en moles de composés de formule (I) où A est un groupe de formule -(CH₂)₄-,
- de 45% à 75% en moles de composés de formule (I) où A est un groupe de formule -(CH₂)₃-, et
- de 15% à 45% en moles de composés de formule (I) où A est un groupe de formule -(CH₂)₂- ;
   le nombre total de moles des trois types de composés étant de 100% ; et
- au moins 10% en poids des groupes R¹ et/ou R² par rapport à la totalité de groupes R¹ et R² sont des groupes isoamyle.

Les compositions de matières de l'invention sont particulièrement efficaces, notamment, pour éliminer des matières de type pétrole. Ils permettent d'éliminer plus de telles matières et/ou d'éliminer autant de matière mais plus rapidement.

Ils présentent de plus un excellent profile écologique, un excellent profile de sécurité d'utilisation, et permettent une excellente optimisation/économie des ressources disponibles sur terre, notamment des bio ressources.

L'invention concerne aussi un procédé de préparation des compositions de matière de l'invention.

L'invention concerne également l'utilisation comme solvant, co-solvant, décapant, inhibiteur de cristallisation, agent nettoyant, et/ou agent de dégraissage des compositions de matière de l'invention. Ces compositions de matière sont par la suite dénommés "compostions de matière utiles".

L'invention concerne aussi un procédé de préparation des compositions de matières utiles.

L'invention concerne aussi des compositions d'application comprenant les compositions de matières utiles. L'invention concerne aussi des procédés dans lesquels on met en oeuvre une solvatation, une co-solvatation, un décapage, une inhibition de cristallisation, un nettoyage, et/ou un dégraissage, en présence des compositions de matière utiles. Le procédé complet peut notamment inclure la phase de préparation desdits compositions de matière.

Les compositions de matières utiles, sont particulièrement efficaces, notamment, pour éliminer des matières de type pétrole ou asphaltènes. Ils permettent d'éliminer plus de telles matières et/ou d'éliminer autant de matière mais plus rapidement.

Ils présentent de plus un excellent profile écologique, un excellent profile de sécurité d'utilisation, et permettent une excellente optimisation/économie des ressources disponibles sur terre, notamment des bio ressources.

### Définitions

Dans la présente demande un composé désigne une substance chimique présentant une formule chimique unique, lorsqu'il est isolé (avec une pureté supérieure à 99% en poids).

Dans la présente demande une composition de matière désigne un mélange de plusieurs composés. Une composition de matière peut être définie en mentionnant tout ou partie de chacune des formules chimiques précises de composés qu'elle comprend, ou en mentionnant tout ou partie de plusieurs formules chimiques générales regroupant plusieurs composés (familles de composés), utilisant le cas échéant des moyennes, ou en mentionnant une formule générale unique avec des moyennes. Une composition de matière comprend au moins 50% en poids de composés répondant aux formules chimiques (formules précises, ou formule(s) générale(s), ou formule(s) moyenne(s)) utilisées pour les définir, de préférence au moins 75%, de préférence au moins 90%, de préférence au moins 99%.

Dans la présente demande une famille de composés désigne un groupe de composés présentant au moins une caractéristique structurelle commune, par exemple un group identique. Il peut s'agir d'un sous groupe d'une composition de matière. On peut par exemple faire référence à une famille dont le nombre d'atomes de carbone du groupe A est de 3, à une famille où il est de 4 etc, à une famille où la groupe A est branché, et/ou à une famille où les groupes R¹ et/ou R² comprennent un groupe isoamyle etc...

Dans la présente demande, la moyenne en nombre d'un nombre d'atomes dans une composition de matière est donnée par la formule *moyenne* = ∑*xᵢNᵢ* où:
- xᵢ est la fraction molaire du composé considéré ou d'une famille considérée
- Nᵢ est le nombre d'atomes considérés dans le composé considéré ou la famille considérée (une famille considérée regroupe le même nombre d'atomes considérés).

Si un nombre moyen est égal à un entier, il peut s'agir d'un composé unique ou d'une famille unique de composé, présentant ce nombre d'atomes, ou il peut s'agir d'une composition de matière comprenant plusieurs composés ou familles de composés avec des nombre d'atomes différents, dans des proportions telles que le nombre moyen est égal à l'entier.

### Composition de matière de formule (I)

La composition de matière de l'invention présente la formule suivante (I):

R¹-OOC-A-COO-R² (I)

où
- les groupes R¹ et R², identiques ou différents, représentent un groupe alkyle, aryle, alkyaryl, ou arylalkyle, linéaire ou branché, cyclique ou non cyclique, en C₁-C₂₀
caractérisé(e) en ce que:
- au moins une partie des groupes R¹ et/ou R² sont des groupes alkyl branchés comprenant 5 atomes de carbone, et/ou
- au moins une partie des groupes R¹ et/ou R² proviennent d'huile de fusel,
- la composition de matière comprenant :
   - de 1% à 20% en moles de composés de formule (I) où A est un groupe de formule -(CH₂)₄-,
   - de 45% à 75% en moles de composés de formule (I) où A est un groupe de formule -(CH₂)₃-, et
   - de 15% à 45% en moles de composés de formule (I) où A est un groupe de formule -(CH₂)₂- ;
      le nombre total de moles des trois types de composés étant de 100% ; et
   - au moins 10% en poids des groupes R et/ou R par rapport à la totalité de groupes R¹ et R² sont des groupes isoamyle.

La composition de matière peut correspondre à un produit de réaction complexe, où des mélanges de réactifs sont utilisés. Par exemple la réaction d'un mélange de HOOC-A^{a}-COOH et HOOC-A^{b}-COOH avec un alcool R^{a}-OH peut donner un mélange des produits R^{a}OOC-A^{a}-COOR^{a} et R^{a}OOC-A^{b}-COOR^{a}. De même la réaction de HOOC-A^{a}-COOH avec un mélange d'alcools R^{a}-OH et R^{b}-OH peut donner un mélange des produits R^{a}OOC-A^{a}-COOR^{a} et R^{b}OOC-A^{a}-COOR^{b}, R^{a}OOC-A^{a}-COOR^{b}, et R^{b}OOC-A^{a}-COOR^{a} (différent de R^{a}OOC-A^{a}-COOR^{b} si A^{a} n'est pas symétrique). De même la réaction d'un mélange de HOOC-A^{a}-COOH et HOOC-A^{b}-COOH avec un mélange d'alcools R^{a}-OH et R^{b}-OH peut donner un mélange des produits, R^{a}OOC-A^{a}-COOR^{a} et R^{b}OOC-A^{a}-COOR^{b}, R^{a}OOC-A^{a}-COOR^{b}, R^{b}OOC-A^{a}-COOR^{a} (différent de R^{a}OOC-A^{a}-COOR^{b} si A^{a} n'est pas symétrique), R^{a}OOC-A^{b}-COOR^{a} et R^{b}OOC-A^{b}-COOR^{b}, R^{a}OOC-A^{b}-COOR^{b}, R^{b}OOC-A^{b}-COOR^{a} (différent de R^{a}OOC-A^{b}-COOR^{b} si A^{b} n'est pas symétrique). Ainsi dans le cas de produits issus de réactifs de compositions complexes, il peut être approprié de définir en partie les produits par les réactifs ou leur provenance.

Par soucis pratique on peut répartir la description des groupes comme suit:
- d'une part les groupes R¹ et R², pouvant correspondre à des alcools R¹-OH et R²-OH (respectivement). On peut assimiler ces groupes aux alcools
- d'autre part le(s) groupe(s) A, pouvant correspondre à un ou des acide(s) dicarboxylique(s) HOOC-A-COOH. On peut assimiler le(s) groupe(s) A au(x) diacides correspondant (le diacide comprend 2 atomes de carbone de plus que le groupe A).

### Groupes R¹ et R ² - Huile de Fusel

Les groupes R¹ et R² peuvent être identiques ou différents. Comme exposé ci-dessus les groupes R¹ et R² peuvent être identiques si on utilise un réactif unique ou pure. Si on utilise un réactif comprenant plusieurs composés différents correspondant à ces groupes alors on obtient différents composés, dont le mélange correspond à une composition de matière. On peut dans ce cas distinguer des familles comprenant au moins un groupe R¹ donné, des familles comprenant un et un seul groupe R¹ donné, des familles comprenant deux groupes identiques R¹=R² donnés, des familles comprenant des couples de groupes R¹ et R² donnés (ces familles ne s'excluent pas forcement les unes des autres).
Pour la composition de matière,
- au moins une partie des groupes R¹ et/ou R² sont des groupes alkyl branchés comprenant 5 atomes de carbone, et/ou
- au moins une partie des groupes R¹ et/ou R² proviennent d'huile de fusel.

L'huile de fusel, parfois dénommée également alcool de fusel, est un mélange comprenant des alcools dits d'ordre supérieur, à au moins trois atomes de carbone. Il s'agit d'un sous produit de fermentation alcoolique (ethanolique en particulier) de végétaux. L'huile de fusel peut notamment être produite lors de la distillation d'éthanol après une fermentation de végétaux (ceci est opéré par exemple pour la production d'éthanol et/ou pour la production de boissons alcoolisées de type distillées (degré d'alcool supérieur à 50°). On opère par fermentation d'au produit végétal, distillation du produit de fermentation, par exemple dans une colonne à distiller, et récupération de la fraction non volatile par exemple en pied de colonne. Le végétal peut notamment être de la betterave, de la canne à sucre, de la pomme de terre, de la patate douce, un fruit, notamment le raisin, un légume, une céréale, notamment le blé ou l'orge, un oléagineux, le riz, ou un mélange. La fermentation est généralement destinée à produire de l'éthanol, la distillation est généralement destinée à récupérer un produit à fort taux d'éthanol. L'huile de fusel est le résidu de distillation, généralement récupéré en pied de colonne de distillation. Ce résidu est parfois aussi dénommé mélasse. Des procédés de préparation d'huile de fusel sont connus, on se réfère par exemple au document EP624388. La composition de l'huile de fusel peut varier selon les procédés et selon le végétal fermenté. Les différentes compositions sont connues. L'huile de fusel comprend généralement de l'alcool isoamylique (3-méthylbutanol-1-ol), le plus souvent associé à d'autres alcools d'ordre supérieur, à au moins trois atomes de carbone, notamment des alcools linéaires ou branchés en C3, C4, C5 (autres que l'alcool isoamylique), ou C6, et/ou à de l'éthanol. Elle peut notamment comprendre, en plus de l'alcool isoamylique, de l'éthanol, du n-butanol, de isobutanol, de l'alcool n-amylique, du n-propanol. L'huile de fusel peut notamment comprendre d'autres composés, comme de l'eau (en proportions généralement de 1 à 20% en poids, de préférence inférieure à 10% en poids), et des impuretés comme des éthers, des acides gras, des furfurals (dans des proportions typiquement inférieures à 15% en poids, de préférence inférieures à 10%, de préférence inférieures à 5%). Les huiles de fusel sont parfois caractérisées par leur point d'ébullition ou par des fourchettes de points d'ébullition. Celui-ci peut aller de 80° à 150°. Les huiles de fusel dont le point d'ébullition est dans la fourchette 110°C à 130°C (ou moins de 130°C) s'avèrent particulièrement utiles.

On préfère que l'huile de fusel comprennent au moins 10% en poids, de préférence au moins 20% en poids, de préférence au moins 40% en poids, de préférence au moins 50% en poids, et par exemple jusqu'à 70% en poids, par rapport à la totalité des alcools présents dans l'huile de fusel, d'alcool(s) branché(s) comprenant 5 atomes de carbones, de préférence d'alcool isoamylique. L'huile de fusel peut typiquement comprendre également de 5 à 40% en poids d'éthanol, de 1 à 8% en poids de 1-propanol, de 0 à 1% en poids de 2-propanol, de 5 à 15% en poids de 2-méthyl-propanol, de 0 à 1% en poids de 1-butanol, de 10 à 30% en poids de 2-methyl-butanol, par rapport à la totalité des alcools présents dans l'huile de fusel, les proportions étant telles que le total est de 100%.

Au moins une partie des groupes R¹ et/ou R² sont des alkyl branchés comprenant 5 atomes de carbone, et/ou provenant d'huile de fusel. On note que ces deux caractéristiques ne s'excluent pas car l'huile de fusel, comme mentionné plus haut, comprend généralement au moins un alcool branché comprenant 5 atomes de carbone (comme l'alcool isoamylique), de formule R-OH ou R correspond à un tel alkyle.

Les autres groupes R¹ et/ou R² peuvent notamment être des groupes alkyle, aryle, alkyaryl, ou arylalkyle, linéaire ou branché, cyclique ou non cyclique, en C₁-C₂₀, différents de groupes groupes alkyl branchés comprenant 5 atomes de carbone, provenant ou non d'huile de fusel. Il peut notamment s'agir de en C₁-C₈, par exemple des groupes choisis parmi les groupes méthyl, éthyle, n-propyle, isopropyle, n-butyle, isobutyle, n-amyle, n-hexyle, cyclohexyle, 2-ethylhexyle, isooctyle, leurs mélanges.

Selon un mode de réalisation pratique, on met en oeuvre une composition de matière comprenant:
- des composés de formule (I) où R¹ et/ou R² sont des groupes isoamyle, et
- au moins un composé choisi parmi les composés suivants:
   - des composés où R¹ et/ou R² sont des groupes éthyle,
   - des composés où R¹ et/ou R² sont des groupes n-propyle,
   - des composés où R¹ et/ou R² sont des groupes isopropyle,
   - des composés où R¹ et/ou R² sont des groupes n-butyle,
   - des composés où R¹ et/ou R² sont des groupes isobutyle,
   - des composés où R¹ et/ou R² sont des groupes n-amyle,
   - des mélanges de ces composés.

La composition de matière de l'invention présente au moins 10% en poids, par rapport à la totalité de groupes R¹ et R² des composés répondant à la formule (I), de préférence au moins 20%, de préférence au moins 40%, de préférence au moins 50%, de groupes R¹ et/ou R² qui sont des groupes isoamyle. Les quantités peuvent être déterminées par analyses et/ou par les quantités de réactifs mis en oeuvre lors de la préparation du composé ou de la composition de matière. Par exemple si on met en oeuvre un unique alcool branché comprenant 5 atomes de carbone, la proportion en poids des groupes R¹ et R² correspondants sera de 100%. Si on met en oeuvre un mélange comprenant un alcool branché comprenant 5 atomes de carbone et un alcool autre, la proportion en poids de groupes R¹ et/ou R² alkyl branchés comprenant 5 atomes de carbone peut être considérée comme égale à la proportion de l'alcool correspondant dans le mélange d'alcool.

On peut notamment mettre en oeuvre un produit (une composition de matière) susceptible d'être obtenu par réaction d'un diacide de formule HOOC-A-COOH, ou d'un diester de formule MeOOC-A-COOMe ou d'un dichlorure d'acyle de formule ClOC-A-COCl ou d'un imide de formule 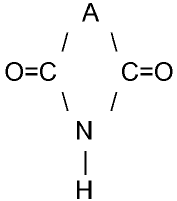 avec de l'huile de fusel, par exemple obtenue comme mentionné plus haut.

### Groupe A

Le groupe A est un groupe alkylène divalent comprenant en moyenne au moins 2,1 atomes de carbone, de préférence de 2,5 à 10 atomes de carbone. Il peut s'agir d'un groupe unique, avec un nombre d'atomes de carbone entier, supérieur ou égal à 3, par exemple égal à 3 ou 4, à l'exclusion d'un produit de réaction d'une huile de fusel ayant un point d'ébullition de 110 à 136°C et d'acide adipique ou sébacique pur. Un tel groupe unique peut correspondre à la mise en oeuvre acide unique. Il peut alternativement s'agir d'un mélange de groupes correspondant à un mélange de composés, dont au moins un présente au moins 3 atomes de carbone. On mentionne que les mélanges de groupes A peuvent correspondre à des mélanges de différents groupes isomères comprenant un nombre identique d'atomes de carbone et/ou de différents groupes comprenant des nombres d'atomes de carbone différents. Le groupe A peut comprendre de groupes linéaires et/ou branchés.

La mise en oeuvre de mélanges de groupes A peut notamment s'avérer avantageuse dans le cadre de certaines utilisations. Les mélanges de groupes A peuvent notamment conférer des profiles de dissolutions et/ou des profiles d'évaporation en fonction du temps et/ou d'une température plus étalés, plus répartis. Des profiles d'évaporation plus étalés ou plus répartis peuvent être intéressants dans le cadre d'application de revêtements en solution dans des compositions de matières solvantes. De tels profiles peuvent notamment améliorer la qualité du revêtement (par exemple son aspect et/ou son adhésion et/ou sa répartition sur le substrat).

La composition de matière comprend (le nombre total de moles des trois types de composés étant de 100%):
- de 1 à 20%, de préférence de 5 à 15%, en moles, de composés de formule (I) où A est un groupe de formule -(CH₂)₄-,
- de 45 à 75%, de préférence de 55 à 65%, en moles, de composés de formule (I) où A est un groupe de formule -(CH₂)₃-, et
- de 15 à 45%, de préférence de 20 à 33%, en moles, de composés de formule (I) où A est un groupe de formule -(CH₂)₂-.

### Compositions de matière particulières

Des compositions de matières particulières de l'invention sont les suivantes:
- Composition comprenant les composés suivants:
   - R_{IA}OOC-(CH₂)₃-COOR_{IA}
   - R_{IA}OOC-(CH₂)₄-COOR_{IA},
   - R_{IA}OOC-(CH₂)₂-COOR_{IA},
   - et éventuellement:
      - R_{IA}OOC-(CH₂)₃-COOR_{Et},
      - R_{IA}OOC-(CH₂)₃-COOR_{Pr},
      - R_{IA}OOC-(CH₂)₃-COOR_{MB},
      - R_{IA}OOC-(CH₂)₄-COOR_{Et},
      - R_{IA}OOC-(CH₂)₄-COOR_{Pr},
      - R_{IA}OOC-(CH₂)₄-COOR_{MB},
      - R_{IA}OOC-(CH₂)₂-COOR_{Et},
      - R_{IA}OOC-(CH₂)₂-COOR_{Pr}, et/ou
      - R_{IA}OOC-(CH₂)₂-COOR_{MB}
   où R_{IA} est un groupe isoamyle,
   R_{MB} est un groupe 2-méthyl-butyle,
   Rₚᵣ est un groupe n-propyle ou isopropyle,
   et R_{EI} est un groupe éthyle.

### Procédés

Les compostions de matière utiles peuvent être préparés par tout procédé approprié. Un procédé pour préparer l'adduit d'acide adipique et d'huile de fusel est par exemple décrit dans le document "The use of Egyptian Fusel Oil for the préparation of some plasticizers compatible with polyvinyl Chloride", Chuiba et al., Indian Journal of Technology, Vol. 23, August 1985, pp 309-311. On peut utiliser un procédé similaire le cas échéant (pour les compositions de matière de l'invention) en remplaçant l'acide adipique par un autre diacide ou par un mélange de diacides de formule HOOC-A-COOH.

Les composés ou compositions de matière peuvent par exemple être obtenus par un procédé comprenant une étape dite d'estérification par réaction d'un diacide de formule HOOC-A-COOH, ou d'un diester de formule MeOOC-A-COOMe ou d'un dichlorure d'acyle de formule ClOC-A-COCl ou d'un imide de formule 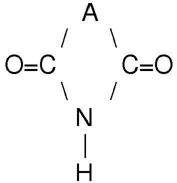 avec:
- un alcool branché comprenant 5 atomes de carbone, ou un mélange d'alcools comprenant un alcool branché comprenant 5 atomes de carbone, de préférence de l'alcool isoamylique ou une composition de matière comprenant de l'alcool isoamylique, et/ou
- de l'huile de fusel.

On peut notamment mettre en oeuvre un mélange de diacides, diesters, dichlorures d'acyle, ou imides.

Les réactions peuvent être catalysées de manière appropriée. On met de préférence en oeuvre au moins 2 équivalents molaires d'alcools par diacide, diester, dichlorure d'acyle, ou imides. On peut par exemple mettre en oeuvre de 2 à 2,5 équivalents pour les réactions avec les diacides, diesters, ou dichlorures d'acyle. On peut par exemple mettre en oeuvre de 5 à 25, par exemple de 10 à 20 équivalents pour les réactions avec les imides. Les réactions peuvent être le cas échéant favorisées par extraction des sous-produits de réaction (par exemple extraction par évaporation du méthanol lors d'une trans-estérification à partir du diester).

La réaction peut être suivie d'étapes de filtration et/ou de purification par exemple par distillation.

L'alcool ou le mélange d'alcools peut être représenté par la formule R-OH, où R représente un groupe R¹, R², ou un mélange de groupes R¹ et R². De tels groupes et alcools correspondants ont été décrits ci-dessus. L'huile de fusel a également fait l'objet d'une description ci-dessus.

Selon un mode utile on opère par réaction avec un diacide ou un mélange de diacides. On peut également opérer par réaction avec un diester dit léger de formule Me-OOC-A-COOMe. Dans ce cas il s'agit plus précisément d'une trans-estérification à partir d'un diesters léger, pour obtenir un diester plus lourd conforme à l'invention. Des diacides ou mélanges de diacides ont été décrits ci-dessus (par le groupe A), et sont disponibles dans le commerce. On peut notamment mettre en oeuvre un mélange d'acide adipique, d'acide glutarique, et d'acide succinique, usuellement dénommé "AGS". Peut mettre en oeuvre pour la réaction de trans-estérification un mélange de diesters légers comme un mélange d'adipate de diméthyle, de glutarate de diméthyle et de succinate de diméthyle, par exemple commercialisé par Rhodia sous le nom Rhodiasolve® RPDE.

Selon un mode particulier, on met en oeuvre comme réactif de l'acide 2-méthylglutarique, ou un mélange d'acide 2-éthylsuccinique, et d'acide 2-méthylglutarique, ou un mélange d'acide 2-éthylsuccinique, d'acide 2-méthylglutarique et d'acide adipique, ou des diesters corresponants. Les diacides sous forme de mélanges, peuvent notamment être obtenus à partir d'un mélange de composés dinitriles notamment produit et récupéré dans le procédé de fabrication de l'adiponitrile par double hydrocyanation du butadiène. Ce procédé utilisé à grande échelle dans l'industrie pour produire la grande majorité de l'adiponitrile consommé dans le monde est décrit dans de nombreux brevets et ouvrages. La réaction d'hydrocyanation du butadiène conduit majoritairement à la formation de dinitriles linéaires mais également à une formation de dinitriles ramifiés dont les deux principaux sont le méthylglutaronitrile et l'éthylsuccinonitrile. Dans les étapes de séparation et de purification de l'adiponitrile, les composés dinitriles ramifiés sont séparés par distillation et récupérés, par exemple, comme fraction de tête dans une colonne de distillation. Les dinitriles ramifiés peuvent par la suite être transformés en diacides ou en diesters (soit en diesters légers pour une réaction ultérieure de trans-estérification avec l'alcool ou le mélange d'alcool ou l'huile de fusel, soit directement en diesters conformes à l'invention).

Un des procédés possibles pour la transformation des dinitriles en diesters correspond à la mise en oeuvre de la réaction de PINNER, notamment décrite dans le brevet français n° 1488857. Sommairement, ce procédé consiste à faire réagir les composés dinitriles avec un alcool en présence d'un acide minéral fort tel que l'acide suflurique, puis à hydrolyser les produits obtenus pour récupérer des diesters par distillation. Ce document décrit également un mode de réalisation particulier du procédé qui consiste à faire passer le mélange de composés dinitriles et l'alcool dans un bain de sels fondus à base de différents sulfates alcalins et ammonium pour éviter la formation de sulfate d'ammonium et récupérer l'ammoniaque par extraction à la vapeur d'eau. Des diesters utiles peuvent également être obtenus par réaction entre les composés dinitriles, de l'eau, et un alcool en phase gaz et en présence d'un catalyseur solide. La température de réaction est avantageusement supérieure à la température de condensation des diesters formés. Comme catalyseur, on peut utiliser un catalyseur solide acide tel que par exemple un gel de silice, un mélange silice-alumine, des acides boriques ou phosphoriques supportés. On peut également utiliser des alumines macroporeuses telles que celles décrites dans le document EP805801. La température de réaction de transformation de dinitriles en diesters peut être comprise entre 200°C et 450°C, de préférence entre 230°C et 350°C. La réaction peut être réalisée sous une pression quelconque, avantageusement comprise entre 0,1 et 20 bar. En sortie de réacteur les vapeurs peuvent être refroidies rapidement à une température inférieure ou égale à 150°C. Du mélange obtenu, on peut séparer par distillation l'ammoniac, puis l'eau et l'alcool en excès. Des diesters utiles peuvent également être obtenus par réaction entre les composés dinitriles et une base minérale, pour obtenir des sels d'acide, puis neutralisation de ces sels par un acide, suivie d'une estérification avec un alcool. Un procédé utile est notamment détaillé dans la demande de brevet français déposée le 9 juin 2006 sous le n° 06 05119 et publiée sous le numéro FR 2 902 095.

Des diacides utiles peuvent être obtenus par réaction entre les composés dinitriles et une base minérale, pour obtenir des sels d'acide, puis neutralisation de ces sels par un acide. Des diacides utiles peuvent également être obtenus par hydrolyse acide des composés dinitriles. Des détails quant à de telles réactions sont donnés dans les documents suivants:
- demande de brevet publiée sous le n° WO2007/101929,
- demande de brevet en France déposée le 09 Juin 2006 sous le n° 0605119, publiée sous le numéro FR 2 902 095 ;
- demande de brevet en France déposée le 24 Novembre 2006 sous le n° 0610302, publiée sous le numéro FR 2 909 088.

Selon un mode particulier, on met en oeuvre un réactif imide ou mélange d'imides. La réaction d'estérification peut être conduite en phase gazeuse, à température élevée par exemple de 250 à 300°C, le cas échéant en présence d'un catalyseur comme du TiO₂, du ZrO₂ ou du CeO₂. L'imide ou le mélange d'imide peut notamment être obtenu à partir d'un dinitrile ou d'un mélange de dinitriles par une réaction dite d'imidification par réaction d'un dinitrile ou d'un mélange de dinitriles de formule NC-A-CN avec de l'eau en phase gazeuse, le cas échéant en présence d'un catalyseur comme du TiO₂. La réaction d'estérification peut être subséquente ou simultanée à la réaction d'imidification. A titre d'imides, on peut notamment utiliser le 2-méthylglutarimide, l'éthylsuccinimide, les mélanges de 2-méthylglutarimide et d'éthylsuccinimide, les mélanges de 2-méthylglutarimide, d'éthylsuccinimide et d'adipimide. Des détails quant aux réactions où des imides ou mélanges d'imides sont mis en oeuvre, sont donnés dans la demande de brevet PCT déposée le 05 Juillet 2007, sous le n° PCT/FR2007/001140, publiée sous le numéro WO2008/009792.

### Utilisations

Les compositions de matière utiles peuvent notamment être utilisés comme solvants, co-solvants, décapants, inhibiteur de cristallisation, agents nettoyants, et/ou agent de dégraissage.

Par co-solvant, on entend que d'autres solvants peuvent lui être associés. L'utilisation à titre de solvant ou de co-solvant comprend notamment des utilisation pour dissoudre un composé dans une formulation, dans un milieu réactionnel, l'utilisation pour solubiliser totalement ou partiellement un produit à éliminer (dégraissage, décapage), et/ou pour faciliter de décollage de films de matières.

Les composés ou compositions de matière utiles peuvent notamment être utilisés, pour les fonctions indiquées ci-dessus ou pour d'autres, dans une formulation phytosanitaire, dans une formulation du nettoyage, dans une formulation de décapage, dans une formulation de dégraissage, dans une formulation de nettoyage, dans une formulation de lubrifiants, dans une formulation de revêtement, dans une formulation de pigments ou encre, dans une formulation plastique.
Les formulations de nettoyage et/ou de dégraissage peuvent notamment être des formulations pour les soins ménagers, opérés dans les foyers ou dans les domaines publiques (hotels, bureaux, usines....). Il peut s'agir de formulation pour le nettoyage des surfaces dures comme les sols, les surfaces d'ameublement et d'équipement des cuisines et salles de bain, la vaisselle. Ces formulations peuvent également être utilisées dans la sphère industrielle pour dégraisser des produits manufacturés et/ou les nettoyés.

Les compositions de matière utiles peuvent notamment être utilisés pour retirer une souillure, un revêtement ou un agent d'aide à la fabrication, sur un substrat. Il peut par exemple s'agir de retirer de la peinture, de retirer un reste de moule en plastique utilisé en fonderie, de retirer des graffitis, de retirer des restes de lubrifiants ou film utilisés pour fabriquer ou protéger des objets manufacturés, en métal notamment. Il peut s'agir de retirer un produit, par exemple un revêtement d'outils, utilisés pour appliquer un produit. Le produit restant sur l'outil peut d'ailleurs être considérés comme une souillure. Il peut notamment de bitume à retirer d'outils d'applications ou de moyen de stockage et/ou de transport. La souillure peut notamment être du pétrole brut ou des asphaltènes. Pour ces souillures, le substrat peut par exemple être un rivage pollué que l'on cherche à nettoyer, un textile, une surface sur un site d'extraction de pétrole et/ou de gaz (une plateforme pétrolière par exemple), que l'on cherche à nettoyer pour des raisons d'hygiène et/ou de sécurité.

Les compositions de matière utiles peuvent notamment être utilisés dans des formulations phytosanitaires comprenant un produit actif solide.
D'autres détails ou avantages pourront apparaitre au vu des exemples qui suivent sans caractère limitatif.

### EXEMPLES

On utilise notamment les matières premières suivantes:
- Huile de Fusel 1: Huile de fusel disponible auprès de la société WAKO à point d'ébullition 110-130°C et de densité 0,810-0,850.
- Alcool Isoamylique: Prolabo
- AGS: Mélange comprenant de l'acide adipique (12.5% en moles), de l'acide glutarique (62.5% en moles) et de l'acide succinique (24.5% en moles)
- MGA: Mélange comprenant de l'acide 2-methyl-glutarique (86% en moles), de l'acide Ethyl-succinique (11% en moles) et de l'acide adipique (3% en moles)
- MGI: Mélange comprenant du 2-méthylglutarimide (88.5% en moles) et de l'éthylsuccinimide (11.5% en moles)

### Exemple 1 - Préparation de compositions de matière issues de l'estérification d'une huile de fusel et d'AGS. "Isoamyl d'AGS".

### Exemple 1.1: estérification en présence d'acide sulfurique.

Dans un réacteur de 1L, on introduit successivement l'huile de fusel 1 (280g) puis l'AGS (200g) et 2 g d'acide sulfurique. On chauffe en agitant le mélange réactionnel. Pendant le chauffage de l'eau est distillée en entrainant un peu d'alcools légers résiduels. Le milieu réactionnel est maintenu à 120°C pendant 3 heures. A la fin des 3 heures de chauffage, la température du milieu est ramenée à la température ambiante. Le milieu est alors lavé avec une solution aqueuse d'hydrogénocarbonate de sodium. La phase organique contient 92% de diesters. Elle est ensuite distillée à 180-190°C sous pression de 20 mm de Hg, pour obtenir un produit comprenant 99-100% en poids de diesters.

### Exemple 1.2: estérification en présence d'argile

On opère selon l'exemple 1.1 mais en utilisant 5 g d'argile K10 de (SUD CHEMIE) à la place de l'acide sulfurique. La réaction est conduite à 130°C pendant 3h30. Le milieu réactionnel est ensuite filtré à 80°C sous 1 bar pour éliminer l'argile. La phase organique contient alors 85% de diesters. Elle est ensuite distillée à 180-190°C sous pression de 20 mm de Hg, pour obtenir un produit comprenant 99-100% en poids de diesters.

### Exemple 1.3: estérification en présence de résine AMBERLYST 15

On opère selon l'exemple 1.1 mais en utilisant 5 g de résine AMBERLYST 15 à la place de l'acide sulfurique. La réaction est conduite à 130°C pendant 3h 30 .Le milieu réactionnel est ensuite filtré à 80°C sous 1 bar pour éliminer la résine. La phase organique contient alors 85% de diesters. Elle est ensuite distillée à 180-190°C sous pression de 20 mm de Hg, pour obtenir un produit comprenant 99-100% en poids de diesters.

### Exemple 1.4: estérification en présence de zéolithe HY 720 .

On opère selon l'exemple 1.1 mais en utilisant 5 g de zéolithe HY 720 à la place de l'acide sulfurique. La réaction est conduite à 130°C pendant 3h 30. Le milieu réactionnel est alors filtré à 80°C sous 1 bar pour éliminer la zéolithe. La phase organique contient alors 89% de diesters. Elle est ensuite distillée à 180-190°C sous pression de 20 mm de Hg, pour obtenir un produit comprenant 99-100% en poids de diesters.

### Exemple 2 - Préparation d'une composition de matière issue d'une huile de fusel et de MGA.

Comme précédemment, on adapte la quantité d'huile de fusel en fonction de sa qualité et de l'indice d'acide du MGA utilisé. Dans tous les cas, on utilise un excès d'huile de fusel (2,2 à 2,4 moles d'huile de Fusel pour 1 mole de MGA) de façon à obtenir une conversion quasi complète du MGA.

### Exemple 2.1: estérification en présence d'acide sulfurique.

Dans un réacteur de 1L, on introduit successivement l'huile de fusel 1 (280 g) puis le MGA (210g) et 2 g d'acide sulfurique. On chauffe en agitant le mélange réactionnel. Pendant le chauffage de l'eau est distillée en entrainant un peu d'alcools légers résiduels. Le milieu réactionnel est maintenu à 120°C pendant 3 heures. A la fin des 3 heures de chauffage, la température du milieu est ramenée à la température ambiante. Le milieu est alors lavé avec une solution aqueuse d'hydrogénocarbonate de sodium. La phase organique contient 93 % de diesters. Elle est ensuite distillée à 180-190°C sous pression de 20 mm de Hg, pour obtenir un produit comprenant 99-100% en poids de diesters.

### Exemple 2.2: estérification en présence de zéolithe HY 720

On opère selon l'exemple 2.1 mais en utilisant 5g de zéolithe HY720 à la place de l'acide sulfurique. La réaction est conduite à 130°C pendant 3h 30 . Le milieu réactionnel est ensuite filtré à 80°C sous 1 bar pour éliminer la zéolithe. La phase organique contient alors 88 % de diesters. Elle est ensuite distillée à 180-190°C sous pression de 20 mm de Hg, pour obtenir un produit comprenant 99-100% en poids de diesters.

### Exemple 2.3: estérification en présence d'argile K10

On opère selon l'exemple 2.1 mais en utilisant 5 g d'argile K10 de (SUD CHEMIE) à la place de l'acide sulfurique. La réaction est conduite à 130°C pendant 3h30. Le milieu réactionnel est ensuite filtré à 80°C sous 1 bar pour éliminer l'argile. La phase organique contient alors 87% de diesters. Elle est ensuite distillée à 180-190°C sous pression de 20 mm de Hg, pour obtenir un produit comprenant 99-100% en poids de diesters.

### Exemple 2.4: estérification en présence de résine Amberlyst 15

On opère selon l'exemple 2.1 mais en utilisant 5 g de résine AMBERLYST 15 à la place de l'acide sulfurique. La réaction est conduite à 130°C pendant 3h 30. Le milieu réactionnel est ensuite filtré à 80°C sous 1 bar pour éliminer la résine. La phase organique contient alors 85% de diesters. Elle est ensuite distillée à 180-190°C sous pression de 20 mm de Hg, pour obtenir un produit comprenant 99-100% en poids de diesters.

### Exemple 3 - Préparation d'une composition de matière issue d'une huile de fusel et de MGI

### Exemple 3.1 - Réaction en phase liquide - catalyseur acide TiO₂ Anatase

Dans un réacteur de 300 ml en inox résistant à la pression, on introduit 25 g de MGI, on ajoute 50 g de l'huile de Fusel 1 et 1 g d'oxyde de titane anatase (source: Millenium). On ferme le réacteur et on chauffe en agitant le milieu réactionnel à 250°C. Le réacteur est purgé toutes les heures pour éliminer le gaz ammoniac formé. Apres 5 heures de réaction, la conversion des imides est complète. Le milieu réactionnel est filtré pour récupérer le catalyseur et on distille le filtrat pour séparer l'huile de fusel en excès et le mélange de diesters. Le mélange de diesters est ensuite distillé à 180-190°C sous pression de 20 mm de Hg.

### Exemple 3.2 - Réaction en phase liquide - catalyseur acide TiO₂ Anatase

On opère comme dans l'exemple 3.1 mais en utilisant un réacteur équipé d'un dispositif de purge continu d'ammoniac. Apres 4 h de réaction, on obtient un mélange de diesters

### Exemple 3.3 - réaction en phase gazeuse - catalyseur acide TiO₂ Anatase

Dans un réacteur tubulaire de 30mm de diamètre, on introduit 20 ml d'oxyde de titane anatase sous forme d'extrudé (source: Engelhart). On ajoute sur le dessus du catalyseur 10 ml de poudre de verre comme vaporiseur - mélangeur statique. On chauffe ce lit catalytique à 250°C sous un courant d'azote de 3 L/h. Apres un conditionnement de 2 heures dans ces conditions, on introduit à un débit de 3 ml/ heure une solution de MGI à 20% p/p dans l'huile de fusel 1. Les gaz sont ensuite condensés dans un récepteur plongé dans un bain de glace. Apres 20 heures de réaction, on obtient un mélange de diesters.

### Exemple 3.4 - Réaction en phase liquide - catalyseur acide TiO₂ Anatase

Dans un réacteur de 300 ml en inox résistant à la pression, on introduit 25 g de MGI, on ajoute 200 g d'alcool isoamylique de Prolabo et titrant 99% de pureté, et 1 g d'oxyde de titane anatase (source: Millenium). On ferme le réacteur et on chauffe en agitant le milieu réactionnel à 250°C. Le réacteur est purgé toutes les heures pour éliminer le gaz ammoniac formé. Apres 5 heures de réaction, la conversion du MGI est pratiquement complète. Le milieu réactionnel est filtré pour récupérer le catalyseur et on distille le filtrat pour séparer l'huile de fusel en excès et le mélange de diesters. Le mélange de diesters distillé dans la plage 160 -200°C sous pression de 20 mm Hg.

### Exemple 4 - Utilisation pour la dissolution de pétrole

### Produits utilisés:

- Pétrole Brut lourd - origine Brésil
- Solvants:
   - "DIB": Rhodiasolv® DIB, commercialisé par Rhodia
   - "Fusel AGS": Produit issu de l'exemple 1.1.

### Procédure

1 - Préparation d'une solution mère à 0,67% en pétrole brut du Brésil dans du toluène: 0.530 g de pétrole brut est solubilisé avec 79 g de toluène à température ambiante.
2 - Préparation des flacons d'essai de suivi de cinétique:
   Dans des flacons en verre de 40 ml, on incorpore 4 g de la solution mère préparée en 1. Ces flacons sont placés à l'étuve à 80°C pendant une nuit. Après évaporation du toluène, il reste sous forme de film mince au fond des flacons l'équivalent de 27 mg de pétrole brut.
3 - Suivi de la vitesse de dissolution par mesure de transmittance optique
   La mesure de transmission optique est effectuée à une longueur d'onde de 600 nm au moyen d'une phototrode Metrohm relié à un photomètre.

On place un barreau magnétique au fond du flacon contenant le film mince de pétrole brut (préparation 4-2). L'ensemble est placé sur un agitateur magnétique, à température ambiante.

Le temps «zéro» de l'acquisition du signal de transmittance optique est déclenché lorsqu'on verse dans le flacon contenant les 27 mg de pétrole lourd 30 ml du solvant à étudier.

### Résultat

Plus la transmittance est faible, plus la proportion de pétrole lourd dissous est élevée. Le tableau 1 reporte la transmittance (en %) en fonction du temps.

Le produit de l'invention permet une dissolution plus importante et plus rapide.

**Tableau 1**

| | 0 | 1 min | 1,5 min | 2 min | 3 min | 4 min | 5 min |
|---|---|---|---|---|---|---|---|
| DIB (comparatif) | 100 | 15,5 | 11,7 | 10 | 9,8 | 9,6 | 9,5 |
| Fusel AGS | 100 | 12 | 9,5 | 8 | 6,5 | 6 | 6 |

Il apparait que le produit conforme à l'invention permet une dissolution plus rapide.

### Exemple 5 - Utilisation pour la dissolution d'asphaltènes

### Produits utilisés:

- Asphaltène (fraction de pétrole brut riche en aromatiques)
- Solvants:
   - "DIB": Rhodiasolv® DIB, commercialisé par Rhodia
   - "IPG": mélange de glycérol et d'acétone
   - "MIPG": mélange de glycérol et de MIBK (méthylisobutylcétone)
   - "Fusel AGS": Produit issu de l'exemple 1.1.

On mélange 27 mg d'asphaltènes et 30 mL de solvant. On reporte ci-dessous la transmittance après 12 heures à 50°C, obtenue avec différents solvants:
- DIB (Comparatif): 28%
- Fusel AGS: 1%
- IPG (comparatif): 70%
- MIPG (comparatif): 60%
- Toluène (comparatif): 0,2%

Il apparait que le produit conforme à l'invention permet une excellente dissolution, du même ordre que celle obtenue avec du toluène dont le profile éco-toxicologique est moins bon.

### Exemple 6 - Utilisation pour le nettoyage/dégraissage de surfaces métalliques

### Produits utilisés:

- Produit à nettoyer:
   - Huile Paraffinique: Catanex T121 commercialisée par Shell
   - Pétrole Brut lourd - origine Brésil
- Solvant: "Fusel AGS": Produit issu de l'exemple 1.1.

### Procédure

Sur une plaque en acier on dépose à température ambiante de l'huile paraffinique, de manière à recouvrir toute la plaque. On laisse reposer 24h à température ambiante. Sur une autre plaque en acier on dépose de même du pétrole, mais à 50°C. On laisse reposer 24h en étuve à 50°C.

On trempe chacune des plaques dans le solvant, à température ambiante pour la plaque avec de l'huile paraffinique, à 50°C pour la plaque avec du pétrole.

Toutes les 30 secondes, on sort les plaques de la solution nettoyante, on les passe sous un mince filet d'eau froide (2 litres/minute), et on observe visuellement la surface relative de film d'eau, par rapport à la totalité de la surface de la plaque, restant sur les plaques au bout de 1 minute à l'air libre. Cette surface relative est considérée comme étant le pourcentage d'huile ou de pétrole extrait.

### Résultats

Sur la plaque portant l'huile paraffinique, on observe que 80% de l'huile a été extraite après 4 minutes.

Sur la plaque portant le pétrole, 100% du pétrole a été extrait après 1 minute.

## Revendications

1. Composition de matière à base de diester(s) d'acide(s) dicarboxylique(s) de formule (I) :
R¹-OOC-A-COO-R² (I)
où
- les groupes R¹ et R², identiques ou différents, représentent un groupe alkyle, aryle, alkyaryle, ou arylalkyle, linéaire ou branché, cyclique ou non cyclique, en C₁-C₂₀
caractérisé(e) en ce que :
- au moins une partie des groupes R¹ et/ou R² sont des groupes alkyl branchés comprenant 5 atomes de carbone, et/ou
- au moins une partie des groupes R¹ et/ou R² proviennent d'huile de fusel,
- la composition de matière comprenant :
- de 1% à 20% en moles de composés de formule (I) où A est un groupe de formule -(CH₂)₄-,
- de 45% à 75% en moles de composés de formule (I) où A est un groupe de formule -(CH₂)₃-, et
- de 15% à 45% en moles de composés de formule (I) où A est un groupe de formule -(CH₂)₂- ;
le nombre total de moles des trois types de composés étant de 100% ; et
- au moins 10% en poids des groupes R¹ et/ou R² par rapport à la totalité de groupes R¹ et R² sont des groupes isoamyle.

2. Composition selon la revendication 1, comprenant en outre au moins un des composés suivants :
- R_{IA}OOC-(CH₂)₃-COOR_{Et},
- R_{IA}OOC-(CH₂)₃-COOR_{Pr},
- R_{IA}OOC-(CH₂)₃-COOR_{MB},
- R_{IA}OOC-(CH₂)₄-COOR_{Et},
- R_{IA}OOC-(CH₂)₄-COOR_{Pr},
- R_{IA}OOC-(CH₂)₄-COOR_{MB},
- R_{IA}OOC-(CH₂)₂-COOR_{Et},
- R_{IA}OOC-(CH₂)₂-COOR_{Pr}, et
- R_{IA}OOC-(CH₂)₂-COOR_{MB}
R_{IA} étant un groupe isoamyle,
R_{MB} étant un groupe 2-méthyl-butyle,
R_{Pr} étant un groupe n-propyle ou isopropyle, et
R_{Et} étant un groupe éthyle.

3. Composition selon l'une des revendications précédentes, **caractérisée en ce que** le(s) diester(s) est(sont) un(des) produit(s) obtenu(s) par réaction d'un diacide de formule HOOC-A-COOH, ou d'un diester de formule MeOOC-A-COOMe ou d'un dichlorure d'acyle de formule ClOC-A-COCl ou d'un imide de formule : 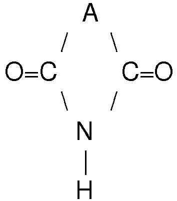 avec de l'huile de fusel.

4. Composition selon la revendication précédente, **caractérisée en ce que** l'huile de fusel est préparée par fermentation d'un végétal, distillation du produit de fermentation, dans une colonne à distiller, et récupération de la fraction non volatile en pied de colonne.

5. Composition selon la revendication précédente, **caractérisée en ce que** le végétal est de la betterave, de la canne à sucre, de la pomme de terre, de la patate douce, un fruit, notamment le raisin, un légume, une céréale, notamment le blé, un oléagineux, le riz, ou un mélange.

6. Procédé de préparation d'une composition selon l'une des revendications précédentes, comprenant une étape dite d'estérification par réaction d'un diacide ou d'un mélange de diacides de formule HOOC-A-COOH, ou d'un diester ou d'un mélange de diesters de formule MeOOC-A-COOMe ou d'un dichlorure d'acyle ou d'un mélange de dichlorure d'acyle de formule ClOC-A-COCl ou d'un imide ou d'un mélange d'imides de formule 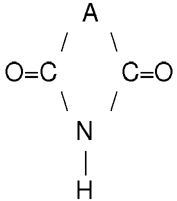 avec :
- un alcool branché comprenant 5 atomes de carbone, ou un mélange d'alcools comprenant un alcool branché comprenant 5 atomes de carbone, et/ou
- de l'huile de fusel.

7. Procédé selon la revendication 6, **caractérisé en ce que** l'imide ou le mélange d'imides est préparé par une réaction dite d'imidification par réaction d'un dinitrile ou d'un mélange de dinitriles NC-A-CN avec de l'eau, la réaction d'estérification étant subséquente à la réaction d'imidification ou simultanée.

8. Utilisation comme solvant, co-solvant, décapant, inhibiteur de cristallisation, agent nettoyant, et/ou agent de dégraissage d'une composition de matière selon l'une quelconque des revendications 1 à 5.

9. Utilisation selon la revendication 8 pour retirer une souillure, un revêtement ou un agent d'aide à la fabrication, sur un substrat.

10. Utilisation selon la revendication 8, **caractérisée en ce que** la souillure est du pétrole brut ou des asphaltènes.

## Patentansprüche

1. Stoffzusammensetzung auf der Basis von Dicarboxylsäurediester(n) mit der Formel (I):
R¹-OOC-A-COO-R² (I),
wobei
- die Gruppen R¹ und R², identisch oder verschieden, eine C₁-C₂₀-Alkyl-, Aryl-, Alkylaryl- oder Arylalkylgruppe, linear oder verzweigt, cyclisch oder nicht cyclisch, darstellen,
**dadurch gekennzeichnet, dass**:
- mindestens ein Teil der Gruppen R¹ und/oder R² verzweigte Alkylgruppen mit 5 Kohlenstoffatomen sind, und/oder
- mindestens ein Teil der Gruppen R¹ und/oder R² von Fuselöl stammt,
- die Stoffzusammensetzung umfasst:
- von 1 Mol-% bis 20 Mol-% Verbindungen mit der Formel (I), wobei A eine Gruppe mit der Formel -(CH₂)₄- ist,
- von 45 Mol-% bis 75 Mol-% Verbindungen mit der Formel (I), wobei A eine Gruppe mit der Formel -(CH₂)₃- ist, und
- von 15 Mol-% bis 45 Mol-% Verbindungen mit der Formel (I), wobei A eine Gruppe mit der Formel -(CH₂)₂- ist;
wobei die Gesamtmolzahl der drei Typen von Verbindungen 100 % beträgt; und
- mindestens 10 Gew.-% der Gruppen R¹ und/oder R² in Bezug auf die Gesamtheit der Gruppen R¹ und R² Isoamylgruppen sind.

2. Zusammensetzung nach Anspruch 1, außerdem umfassend mindestens eine der folgenden Verbindungen:
- R_{IA}OOC-(CH₂)₃-COOR_{Et},
- R_{IA}OOC-(CH₂)₃-COOR_{Pr,}
- R_{IA}OOC-(CH₂)₃-COOR_{MB},
- R_{IA}OOC-(CH₂)₄-COOR_{Et},
- R_{IA}OOC-(CH₂)₄-COOR_{Pr},
- R_{IA}OOC-(CH₂)₄-COOR_{MB},
- R_{IA}OOC-(CH₂)₂-COOR_{Et},
- R_{IA}OOC-(CH₂)₂-COOR_{Pr}, und
- R_{IA}OOC-(CH₂)₂-COOR_{MB},
wobei R_{IA} eine Isoamylgruppe ist,
R_{MB} eine 2-Methylbutylgruppe ist,
R_{Pr} eine n-Propyl- oder Isopropylgruppe ist, und
R_{Et} eine Ethylgruppe ist.

3. Zusammensetzung nach einem der vorhergehenden Ansprüche,
**dadurch gekennzeichnet, dass** der (die) Diester ein Produkt (Produkte) ist (sind), das (die) durch Umsetzen einer Disäure mit der Formel HOOC-A-COOH oder eines Esters mit der Formel MeOOC-A-COOMe oder eines Acyldichlorids mit der Formel ClOC-A-COCl oder eines Imids mit der Formel: 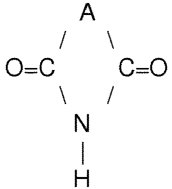 mit Fuselöl erhalten wird (werden).

4. Zusammensetzung nach dem vorhergehenden Anspruch, **dadurch gekennzeichnet, dass** das Fuselöl durch Fermentation einer Pflanze, Destillation des Fermentationsprodukts in einer Distillationssäule und Gewinnung der nichtflüchtigen Fraktion am Fuß der Säule hergestellt wird.

5. Zusammensetzung nach dem vorhergehenden Anspruch, **dadurch gekennzeichnet, dass** die Pflanze eine Rübe, Zuckerrohr, eine Kartoffel, eine Süßkartoffel, ein Obst, insbesondere Trauben, ein Gemüse, Getreide, insbesondere Weizen, eine Ölfrucht, Reis oder eine Mischung ist.

6. Verfahren zur Herstellung einer Zusammensetzung nach einem der vorhergehenden Ansprüche, umfassend einen Schritt, der als Veresterung bezeichnet wird, durch Umsetzen einer Disäure oder einer Mischung von Disäuren mit der Formel HOOC-A-COOH oder eines Diesters oder einer Mischung von Diestern mit der Formel MeOOC-A-COOMe oder eines Acyldichlorids oder einer Mischung von Acyldichlorid mit der Formel ClOC-A-COCl oder eines Imids oder einer Mischung von Imiden mit der Formel: 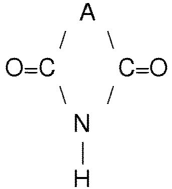 mit:
- einem verzweigten Alkohol mit 5 Kohlenstoffatomen oder einer Mischung von Alkoholen, umfassend einen verzweigten Alkohol mit 5 Kohlenstoffatomen, und/oder
- Fuselöl.

7. Verfahren nach Anspruch 6,
**dadurch gekennzeichnet, dass** das Imid oder die Mischung von Imiden durch eine Reaktion, die als Imidifizierung bezeichnet wird, durch Umsetzen eines Dinitrils oder einer Mischung von Dinitrilen NC-A-CN mit Wasser hergestellt wird, wobei die Veresterungsreaktion nach der Imidifizierungsreaktion oder gleichzeitig erfolgt.

8. Verwendung einer Stoffzusammensetzung nach einem der Ansprüche 1 bis 5, als Lösungsmittel, Co-Lösungsmittel, Ätzmittel, Kristallisationsinhibitor, Reinigungsmittel und/oder Entfettungsmittel.

9. Verwendung nach Anspruch 8, um eine Verunreinigung, eine Beschichtung oder ein Herstellungshilfsmittel von einem Substrat zu entfernen.

10. Verwendung nach Anspruch 8, **dadurch gekennzeichnet, dass** die Verunreinigung Rohöl oder Asphaltene sind.

## Claims

1. Material composition based on dicarboxylic acid diester(s) of formula (I):
R¹-OOC-A-COO-R² (I)
where:
- the R¹ and R² groups, which are identical or different, represent a linear or branched, cyclic or noncyclic, C₁-C₂₀ alkyl, aryl, alkylaryl or arylalkyl group,
**characterized in that**:
- at least a portion of the R¹ and/or R² groups are branched alkyl groups comprising 5 carbon atoms, and/or
- at least a portion of the R¹ and/or R² groups originate from fusel oil,
- the material composition comprising:
- from 1 mol% to 20 mol% of compounds of formula (I) where A is a group of formula -(CH₂)₄-,
- from 45 mol% to 75 mol% of compounds of formula (I) where A is a group of formula -(CH₂)₃-, and
- from 15 mol% to 45 mol% of compounds of formula (I) where A is a group of formula -(CH₂)₂-;
the total number of moles of the three types of compounds being 100%; and
- at least 10% by weight of the groups R¹ and/or R², with respect to all groups R¹ and R², are isoamyl groups.

2. Composition according to Claim 1, additionally comprising at least one of the following compounds:
- R_{IA}OOC-(CH₂)₃-COOR_{Et},
- R_{IA}OOC-(CH₂)₃-COOR_{Pr},
- R_{IA}OOC-(CH₂)₃-COOR_{MB},
- R_{IA}OOC-(CH₂)₄-COOR_{Et},
- R_{IA}OOC-(CH₂)₄-COOR_{Pr},
- R_{IA}OOC-(CH₂)₄-COOR_{MB},
- R_{IA}OOC-(CH₂)₂-COOR_{Et},
- R_{IA}OOC-(CH₂)₂-COOR_{Pr}, and
- R_{IA}OOC-(CH₂)₂-COOR_{MB}
R_{IA} being an isoamyl group,
R_{MB} being a 2-methylbutyl group,
R_{Pr} being an n-propyl or isopropyl group, and
R_{Et} being an ethyl group.

3. Composition according to either of the preceding claims, **characterized in that** the diester or diesters is/are a product or products obtained by reaction of a diacid of formula HOOC-A-COOH or of a diester of formula MeOOC-A-COOMe or of an acyl dichloride of formula ClOC-A-COCl or of an imide of formula: 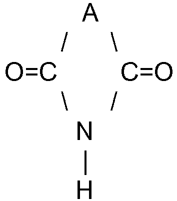 with fusel oil.

4. Composition according to the preceding claim, **characterized in that** the fusel oil is prepared by fermentation of a plant, distillation of the fermentation product, in a distillation column, and recovery of the nonvolatile fraction as column bottoms.

5. Composition according to the preceding claim, **characterized in that** the plant is beet, sugar cane, potato, sweet potato, a fruit, in particular grapes, a vegetable, a cereal, in particular wheat, an oleaginous plant, rice or a mixture.

6. Process for the preparation of a composition according to one of the preceding claims, comprising an "esterification" stage by reaction of a diacid or a mixture of diacids of formula HOOC-A-COOH or of a diester or a mixture of diesters of formula MeOOC-A-COOMe or of an acyl dichloride or a mixture of acyl dichlorides of formula ClOC-A-COCl or of an imide or a mixture of imides of formula 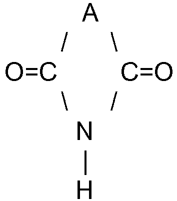 with:
- a branched alcohol comprising 5 carbon atoms or a mixture of alcohols comprising a branched alcohol comprising 5 carbon atoms, and/or
- fusel oil.

7. Process according to Claim 6, **characterized in that** the imide or the mixture of imides is prepared by an "imidation" reaction by reaction of a dinitrile or a mixture of dinitriles NC-A-CN with water, the esterification reaction being subsequent to or simultaneous with the imidation reaction.

8. Use, as solvent, cosolvent, stripper, crystallization inhibitor, cleaning agent and/or degreasing agent, of a material composition according to any one of Claims 1 to 5.

9. Use according to Claim 8, for removing, from a substrate, a contaminant, a coating or an agent for helping in the manufacture.

10. Use according to Claim 8, **characterized in that** the contaminant is crude petroleum or asphaltenes.
